(19) Europäisches Patentamt
European Patent Office
Office européen des brevets

(11) EP 1 264 900 A2

(12) EUROPEAN PATENT APPLICATION

(43) Date of publication:
11.12.2002 Bulletin 2002/50

(51) Int Cl.7: C12Q 1/68, G01N 33/543

(21) Application number: 02012436.8

(22) Date of filing: 10.06.2002

(84) Designated Contracting States:
AT BE CH CY DE DK ES FI FR GB GR IE IT LI LU MC NL PT SE TR
Designated Extension States:
AL LT LV MK RO SI

(30) Priority: 08.06.2001 JP 2001174879

(71) Applicant: FUJI PHOTO FILM CO., LTD.
Kanagawa-ken, 250-0123 (JP)

(72) Inventors:
- Shinoki, Hiroshi
  Asaka-shi, Saitama 351-8585 (JP)
- Sakaino, Yoshiki
  Asaka-shi, Saitama 351-8585 (JP)
- Seshimoto, Osamu
  Asaka-shi, Saitama 351-8585 (JP)

(74) Representative: Albrecht, Thomas, Dr. et al
Kraus & Weisert,
Thomas-Wimmer-Ring 15
80539 München (DE)

# (54) Roll of web having reactive groups on its surface

(57) A roll of flexible web having on its surface a plurality of reactive vinylsulfonyl groups fixed to the web via a linking group is favorably employable for manufacturing analytical elements to be used for analysis of biochemically active substances, for instance, analytical elements to which nucleotide derivatives or their analogues are attached.

EP 1 264 900 A2

## Description

### FIELD OF THE INVENTION

This invention relates to a roll of web having reactive groups on its surface which is favorably employable for preparing analytical elements to be used for analysis of biochemically active substances, for instance, analytical elements to which nucleotide derivatives or their analogues (e.g., oligonucleotides, polynucleotides, and peptide-nucleotides) are attached. The analytical element is generally named DNA chip and is favorably employable for detecting, with high sensitivity, complementary nucleic acid fragments.

### BACKGROUND OF THE INVENTION

Detection of a nucleic acid fragment is generally performed using a probe oligonucleotide which is complementary to the nucleic acid fragment to be detected, by way of hybridization, The probe oligonucleotide is generally fixed onto a solid carrier (e.g., solid substrate) to produce a so-called DMA chip. In the detection procedures, a nucleic acid fragment in a sample liquid is provided with a fluorescent label or a radioisotope label, and then the sample liquid is brought into contact with the probe oligonucleotide of the DNA chip. If the labelled nucleic acid fragment in the sample liquid is complementary to the probe oligonucleotide, the labelled nucleic acid fragment is combined with the probe oligonucleotide by hybridization. The labelled nucleic acid fragment fixed to the DNA chip by hybridization with the probe oligonucleotide is then detected by an appropriate detection method such as fluorometry or autoradiography. The DNA chip is widely employed in the gene technology, for instance, for detecting a complementary nucleic acid fragment and sequencing a nucleic acid.

The DNA chip can be utilized to efficiently detect a large number of complementary nucleic acid fragments in a small amount of a sample liquid almost simultaneously.

Detection of nucleic acid fragment using an electrochemical label is also known (Japanese Patent Provisional Publication No. 9-288080, and a preprint of the 57th Analytical Chemistry Conference pp. 137-138 (1996)).

P.E. Nielsen et al., Science, 254, 1497-1500(1991) and P.E. Nielsen et al., Biochemistry, 36, pp.5072-5077 (1997) describe PNA (Peptide Nucleic Acid or Polyamide Nucleic Acid) which has no negative charge and functions in the same manner as DNA fragment does. PNA has a polyamide skeleton of N-(2-aminoethyl)glycine units and has neither glucose units nor phosphate groups.

Since PNA is electrically neutral and is not charged in the absence of an electrolytic salt, PNA is able to hybridize with a complementary nucleic acid fragment to form a hybrid which is more stable than the hybrid structure given by a probe oligonucleotide and its complementary nucleic acid fragment (Preprint of the 74th Spring Conference of Japan Chemical Society, pp. 1287, reported by Naomi Sugimoto).

Japanese Patent Provisional Publication No.11-332595 describes a PNA probe fixed onto a solid carrier at its one end and a detection method utilizing the PNA probe. The PNA probe is fixed onto the solid carrier by the known combination of avidin and biotin.

The aforementioned P.E. Nielsen et al., Science, 254, 1497-1500(1991) also describes a PNA probe labelled with an isotope element and a detection method of a complementary nucleic acid fragment.

Since the PNA probe shows no electric repulsion to a target nucleic acid fragment in a sample liquid, an improved high detection sensitivity is expected.

At present, two methods are known for preparing a DNA chip having a solid carrier and oligonucleotides or polynucleotides fixed onto the carrier. One preparation method comprises preparing oligonucleotides or polynucleotides step by step on the carrier. This method is named "on-chip method". A typical on-chip method is described in Foder, S.P.A., Science, 251, page 767 (1991).

Another preparation method comprises fixing separately prepared oligonucleotides or polynucleotides onto a solid carrier. Various methods are known for various oligonucleotides and polynucleotides.

In the case that the complementary nucleotide derivatives (which are synthesized using mRNA as mold) or PCR products (which are DNA fragments prepared by multiplying cDNA by PCR method), an aqueous solution of the prepared DNA fragment is spotted onto a solid carrier having a poly-cationic coat in a DNA chip-preparing device to fix the DNA fragment to the carrier via electrostatic bonding, and then blocking a free surface of the polycationic coat.

In the case that the oligonucleotides are synthetically prepared and have a functional group, an aqueous solution of the synthetic oligonucleotides is spotted onto an activated or reactive solid carrier to produce covalent bonding between the oligonucleotides and the carrier surface. See Lamture, J.B., et al., Nucl. Acids Res., 22, 2121-2125, 1994, and Guo, Z., et al., Nucl. Acids Res., 22, 5456-5465, 1994. Generally, the oligonucleotides are covalently bonded to the surface activated carrier via linking groups.

Also known is a process comprising the steps of aligning small polyacrylamide gels on a glass plate and fixing synthetic oligonucleotides onto the glass plate by making a covalent bond between the polyacrylamide and the oligonucleotide (Yershov, G., et al., Proc. Natl. Acad. Sci. USA, 94, 4913(1996)). Sosnowski, R.G., et al., Proc. Natl. Acad. Sci. USA, 94, 1119-1123 (1997) discloses a process comprising the steps of an array of microelectrodes on a silica chip, forming on the microelectrode a streptoavidin-comprising agarose layer, and attaching biotin-modified DNA fragment to the agarose layer by positively charging the agarose layer. Schena, M., et al., Proc. Natl. Acadi Sci. USA, 93, 10614-10619 (1996) teaches a process comprising the steps of preparing a suspension of an amino group-modified PCR product in SSC (i.e., standard sodium chloride-citric acid buffer solution), spotting the suspension onto a slide glass, incubating the spotted glass slide, treating the incubated slide glass with sodium borohydride, and heating thus treated slide glass.

As is explained above, most of the known methods of fixing separately prepared DNA fragments onto a solid carrier utilize the electrostatic bonding or the covalent bonding.

In any DNA chips having separately prepared oligonucleotide probes on its solid carrier, the oligonucleotide probes should be firmly fixed onto the carrier, so that the hybridization can proceed smoothly between the fixed oligonucleotide probes and target DNA fragments complementary to the fixed oligonucleotide probes.

United States Patent No. 5,387,505 describes a method of separating a target DNA fragment by binding target DNA fragments labelled with a biotin molecule with a substrate having avidin molecules.

United States Patent No. 5,094,962 discloses a detection tool for a ligand-receptor assay in which receptor molecules are bonded to a porous polymer particle having a reactive group.

## SUMMARY OF THE INVENTION

It is an object of the present invention to provide a roll of web having on its surface a plurality of reactive groups which are favorably employable for manufacturing solid carriers having biochemically active probe compounds on their surfaces.

The present invention resides in a roll of flexible web having a plurality of reactive groups on a surface thereof, in which the reactive groups are vinylsulfonyl groups that are attached to the flexible web via a linking group. In the roll of flexible web of the invention, the vinylsulfonyl group is preferably represented by the following formula:

$$-SO_2-CR^1=CR^2R^3$$

in which each of $R^1$, $R^2$ and $R^3$ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms. It is preferred that each of $R^1$, $R^2$ and $R^3$ is a hydrogen atom, and the linking group contains -NH-, -S-, or -O-. The linking group containing the group of -NH, -S-, or -O- are preferably provided to the surface of the web by coating the surface with polymer material having a plurality of $-NH_2$, -SH, or -OH, or treating the surface with a silane-coupling agent containing a plurality of $-NH_2$, -SH, or -OH.

In the roll of flexible web of the invention, the reactive groups are preferably attached to the flexible web by reacting divinylsulfone compounds with active hydrogen-containing groups which are previously provided to the flexible web.

The invention further resides in a chip having on a surface thereof a plurality of reactive vinylsulfonyl groups attached to the surface via a linking group which is manufactured by dividing the roll of web of the invention.

The invention furthermore resides in a process for preparing a roll of web having reactive groups on a surface thereof, which comprises bringing divinylsulfone compounds into contact with a surface of a web which has a plurality of active hydrogen-containing groups on the surface, to cause bonding reaction between the hydrogen-containing group and the one vinylsulfonyl group of the divinylsulfone compound, and winding the divinylsulfone compound-bonded web to give a roll.

## DETAILED DESCRIPTION OF THE INVENTION

The flexible web can be a polymer material web. Examples of the polymer materials include cellulose derivatives (e.g., diacetylcellulose, triacetylcellulose, propionylcellulose, butanoylcellulose, and acetylpropionylcellulose), polyamides, polycarbonates, polyesters (e.g., polyethylene terephthalate, polyethylene-1,2-diphenoxyethane-4,4' -dicarboxylate, polybutylene terephthalate, polyethylene naphthalate), polycycloolefins (e.g., Zeonor and Zeonex, tradenames), polyallylate (e.g., U-polymer, tradename), polystyrene, polypropylene, polyethylene, polymethylpentene, polyether sulfone, and polyether amide. Preferred are triacetylcellulose, polyethylene terephthalate, polyethylene naphthalate, and polycycloolefins.

On the flexible web can be provided a metal film such as a film of gold, silver, or copper, or a metal oxide film such as a film of indium titanium oxide or tin oxide. The flexible web can be an electro-conductive flexible metal film.

The flexible web can be previously treated by surface-active treatments such as corona discharge treatment, plasma discharge treatment, glow discharge treatment, laser treatment, ozone-oxidation treatment, surface-roughening treatment, chemical treatment, flame treatment, UV treatment, and high frequency treatment. On thus treated surface or non-treated surface can be placed a layer of polymer material having plural active hydrogen-containing groups such as amino groups ($-NH_2$), mercapto groups (-SH) or hydroxyl groups (-OH). Examples of the polymer materials include poly-L-lysine, polyethyleneimine, polyalkylamine and other amine polymers.

These polymer material can be coated on the web via one or more adhesive layers (undercoat layers). The undercoat layers can be made of a water-soluble polymer such as gelatin, gelatin derivative, casein, agar, sodium alginate, starch, polyvinyl alcohol, polyacrylic acid copolymer, or maleic anhydride copolymer; cellulose ester such as carboxymethylcellulose or hydroxyethylcellulose, or latex polymer such as vinyl chloride copolymer, vinylidene chloride copolymer, acrylate copolymer, vinyl acetate copolymer, or styrene copolymer. The undercoat layer can contain a metal oxide powder such as colloidal silica or alumina powder or other inorganic powder.

Alternatively, the treated or non-treated surface of the web can be treated with a silane-coupling agent having amino groups or other active hydrogen-containing groups. Examples of the amino group-containing silane coupling agents include y-aminopropyltriethoxysilane, N-β-(aminoethyl)-γ-aminopropyltrimethoxysilane, and N-β-(aminoethyl)-γ-aminopropyldimethyldimethoxysilane. Most preferred is γ-aminopropyltriethoxysilane.

The web having on its surface a plurality of active hydrogen-containing groups is brought into contact with divinylsulfone compounds, whereby the active hydrogen-containing groups are reacted with vinylsulfonyl groups of the divinylsulfone compounds, and covalent-bondings are produced.

Accordingly, the vinylsulfonyl group is preferably attached to the surface of the web utilizing a divinylsulfone (or divinylsulfonyl) compound.

The vinylsulfonyl group can have the following formula:

$$-SO_2-CR^1=CR^2R^3$$

In the formula, each of $R^1$, $R^2$ and $R^3$ independently is a hydrogen atom, an alkyl group having 1 to 6 carbon atoms such as methyl, ethyl, n-propyl, isopropyl, n-butyl, or n-hexyl, an aryl group having 6 to 20 carbon atoms such as phenyl or naphthyl, or an aralkyl group having 7 to 26 carbon atoms in which its alkyl group has 1 to 6 carbon atoms such as benzyl or phenethyl. Preferably, each of $R^1$, $R^2$ and $R^3$ is a hydrogen atom.

In place of the divinylsulfone compounds, compounds having precursor groups of the vinylsulfonyl groups may be employed.

Examples of the compounds can be represented by the formula:

$$X^1-SO_2-L^2-SO_2-X^2$$

in which $L^2$ represents a linking group, and each of $X^1$ and $X^2$ represents a group of $-CHR^1-CR^2R^3Y$ wherein each of $R^1$, $R^2$ and $R^3$ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; Y represents a halogen atom, $-SO_2R^{11}$, $-OCOR^{12}$, $-OSO_3M$, or a quaternary pyridinium group; $R^{11}$ is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms; $R^{12}$ is an alkyl group having 1 to 6 carbon atoms, or a halogenated alkyl group having 1 to 6 carbon atoms; and M is a hydrogen atom, an alkali metal atom, or an ammonium group.

Preferred divinylsulfone compounds and analogous compounds are illustrated below:

(S1)

$$H_2C=CH-SO_2-CH_2-SO_2-CH=CH_2$$

(S2)

$$H_2C=CH-SO_2-CH_2OCH_2-SO_2-CH=CH_2$$

(S3)

$$H_2C=CH\text{-}SO_2\text{-}CH_2CH_2CH_2\text{-}SO_2\text{-}CH=CH_2$$

(S4)

$$H_2C=CH\text{-}SO_2\text{-}CH_2CH(OH)CH_2\text{-}SO_2\text{-}CH=CH_2$$

(S5)

$$H_2C=CH\text{-}SO_2\text{-}CH_2CONHCH_2CH_2NHCOCH_2\text{-}SO_2\text{-}CH=CH_2$$

(S6)

$$H_2C=CH\text{-}SO_2\text{-}CH_2CONHCH_2CH_2CH_2NHCOCH_2\text{-}SO_2\text{-}CH=CH_2$$

(S7)

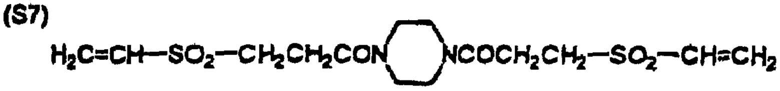

(S8)

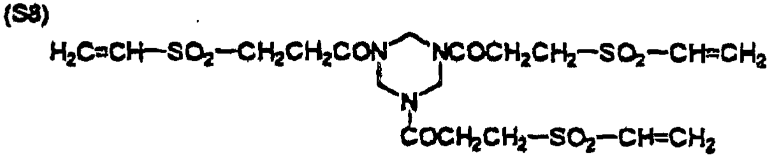

(S9)

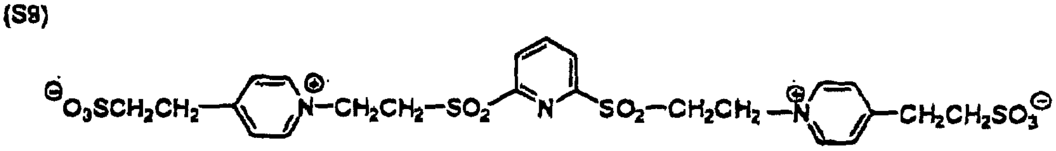

(S10)

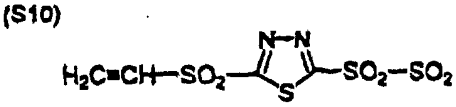

(S11)

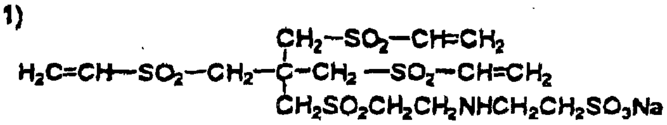

(S12)

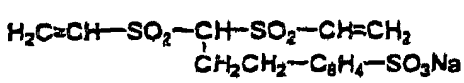

(S13)

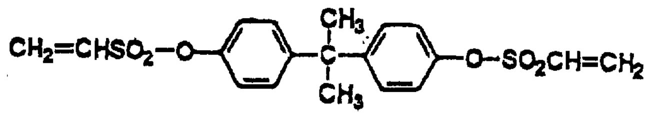

(S14)

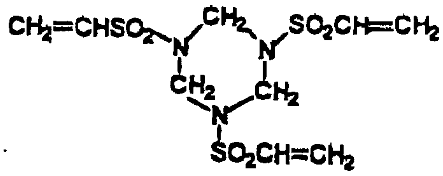

(S15)

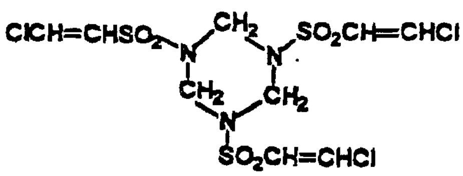

Most preferred are 1,2-bis(vinylsulfonylamide)ethane and 1,2-bis(vinylsulfonylamide)propane.

The contact of the divinylsulfone compound to the web surface can be performed by known coating methods utilizing various coating apparatuses, such as, kiss roll coater, squeeze roll coater, direct gravure roll coater, off-set gravure roll coater, off-set plane roll coater, two roll reverse coater, three roll reverse coater, one roll reverse coater, direct plane roll coater, air knife coater, or dip roll coater. The dip roll coater is preferably employed. In more detail, a solution of divinyl sulfone compound is brought into contact with the web surface utilizing a coating method and then the coated web is dried. The dried web is then wound to give a roll.

The vinylsulfonyl groups attached to the surface of the web are resistant to hydrolysis and highly reactive to probe compounds (such as polynucleotide, oligonucleotide, peptide nucleic acid) having active-hydrogen containing groups such as amino groups ($-NH_2$). Accordingly, the web roll of the invention can be stored for a certain period of time maintaining its reactivity after it is manufactured.

The probe compounds to be fixed to the web can be oligonucleotides, polynucleotides, or peptide-nucleotides. The nucleotide derivatives may be DNA fragments. The nucleotide derivative may be polynucleotide such as cDNA, a portion of cDNA, or EST. The polynucleotide is favorably employed for studying gene expression. Otherwise, nucleotide derivatives to be fixed onto the web may be oligonucleotides, which are favorably employed for studying variations and polymorphism of gene. The oligonucleotide to be fixed onto the web preferably is one of 3 to 50-mers, more preferably 10 to 25 mers. The oligonucleotide and polynucleotide can have one or more substituent groups or cross-linking groups, provided that the attachment of these groups does not impart adverse influence to the function of the oligonucleotide and polynucleotide. For instance, LNA (locked nucleic acid) which is described in J. Am. Chem. Soc., 1998, 120, 13252-13253, can be employed.

The probe compounds to be employed in the invention should have at its one terminal or its vicinity a reactive group which can react with the vinylsulfonyl group or its reactive precursor. The reactive group can be a group of amino, carboxyl, acyl, or carbamoyl. Most preferred is an amino group.

The reactive group can be attached to the nucleotide derivative or its analogue via a linking group. The linking group preferably is an alkylene group or an N-alkylamino-alkylene group. Preferred are a hexylene group and an N-methylamino-hexylene group.

The probe compounds to be fixed onto the web are dissolved or dispersed in an aqueous solution. Generally, the aqueous solution is once placed on a plastic plate having 96 or 384 wells, and then spotted onto a web using a spotting means.

In order to keep the spotted aqueous solution from evaporating, it is preferred to add a high boiling-point compound to the aqueous solution containing probe compounds. The high boiling-point compound should be soluble

in an aqueous medium, should not disturb hybridization procedure, and preferably has an appropriate viscosity. Examples of the high boiling-point compounds include glycerol, ethylene glycol, dimethylsulfoxide, and a hydrophilic polymer having a low molecular weight (typically, in the range of $10^3$ to $10^6$) such as polyacrylamide, polyethylene glycol, or poly(sodium acrylate). The high boiling-point compound preferably is glycerol or ethylene glycol. The high boiling-point compound is preferably incorporated into an aqueous probe compound solution in an amount of 0.1 to 2 vol.%, particularly 0.5 to 1 vol.%. Otherwise, the spotted aqueous solution is preferably kept under the conditions of a high humidity (such as 90%RH or more) and an ordinary temperature (25 to 50°C).

The aqueous solution is spotted onto the web or chip which is obtained by cutting out of the web under the condition that each drop of the solution generally has a volume of 100 pL to 1 µL, preferably 1 to 100 nL. The probe compounds preferably spotted onto the web or chip are in an amount (number) of $10^2$ to $10^5$/cm$^2$. In terms of mol., 1 to $10^{-15}$ moles are spotted. In terms of weight, several ng or less of nucleotide derivatives are spotted. The spotting of the aqueous solution is made onto the web or chip to form several dots having almost the same shape and size. Several dots are formed separately from each other with a distance of 1.5 mm or less, preferably 100 to 300 µm. One dot preferably has a diameter of 50 to 300 µm.

After the aqueous solution is spotted on the web or chip, the spotted solution is preferably incubated, by keeping the web or chip for a certain period at room temperature or under warming, so as to fix the spotted probe compounds onto the web or chip. In the course of incubation, UV irradiation or surface treatment using sodium borohydride or a Shiff reagent may be applied. The UV irradiation under heating is preferably adopted. It is assumed that these treatments are effective to produce additional linkage or bonding between the web or chip and the attached probe compounds. The free (namely, unfixed) probe compounds are washed out using an aqueous solution. Thus washed web or chip is then dried to give a probe compound-fixed web or chip (such as DNA chip).

The present invention is further described by the following examples.

[Example 1 - Manufacture of vinylsulfonyl group-fixed web]

On a transparent, colorless polyethylene terephthalate web (thickness: 180 µm) having a gelatin undercoat layer was coated an aqueous solution (pH: 7.6) containing poly-L-lysine, p-nonylphenoxypolyglycidol, and N-2-hydroxyethylpiperazine-N'-2-ethanesulfonic acid (HEPES) to form a layer having the following coated amounts:

| Coat component | Coated amount (/m$^2$) |
|---|---|
| Poly-L-lysine | 21.5 g |
| p-Nonylphenoxypolyglycidol | 0.8 g |
| N-2-hydroxyethylpiperazine-N'-2-ethanesulfonic acid (HEPES) | 0.3 mg |

The coated web was then dried.

Separately, a phosphate buffer solution (pH 8.5) containing 5 wt.% of 1,2-bis(vinylsulfonylacetamide)-ethane was coated on the coated layer of the web, using a dip roll, and the coated web was dried. The web was then wound to give a roll of web having vinylsulfonyl groups on its surface.

[Example 2] Preparation of chips having vinylsulfonyl groups on their surfaces

The web was re-wounded from the roll prepared in Example 1 and cut by a slitter to give reactive chips (76 mm x 25 mm) having vinylsulfonyl groups on their surfaces.

[Example 3 - Detection of Target Oligonucleotide]

(1) Preparation of DNA chip

An oligonucleotide (3'-TCCTCCATGTCCGGGGAGGATCTGACACTTCAAGGTCTAG-5', 40-mers) having L-glutamylglycine at 3'-terminal was dispersed in 1 µL of an aqueous solution containing a carbonate buffer solution (0.1 M, pH 9.3) at a concentration of 1 x $10^{-6}$ M. The buffer solution was then spotted onto the reactive chip obtained in Example 1, and this was immediately kept at 25°C, 90%RH for one hour. Thus treated chip was then washed successively twice with a mixture of aqueous 0.1 wt.% SDS (sodium dodecylsulfate) solution and aqueous 2xSSC solution (obtained by twice diluting standard sodium chloride-citrate buffer solution (SSC)), and once with the aqueous 0.2xSSC solution. Thus washed chip was placed in an aqueous 0.1 M glycine solution (pH 10) for 1.5 hours, washed with distilled water, and then dried at room temperature, to prepare a DNA chip.

(2) Detection of target oligonucleotide

A target oligonucleotide (CTAGTCTGTGAAGTTCCAGATC-5', 22-mers) having Cy5 (fluorescent label) at its 5'-terminal was dispersed in 20 μL of a hybridizing solution (mixture of 4 x SSC and 10 wt.% SDS). The resulting solution was spotted onto the DNA chip prepared in (1) above, and its spotted surface was covered with a covering glass. Thus covered chip was subjected to incubation at 60°C for 20 hours in a moisture chamber. The incubated chip was washed successively with a mixture of 0.1 wt.% SDS and 2xSSC, a mixture of 0.1 wt.% SDS and 0.2xSSC, and an aqueous 0.2xSSC solution, centrifuged at 700 r.p.m. for 5 minutes, and dried at room temperature.

The fluorescence strength of thus treated DNA chip was measured using a fluorescence scanning apparatus. The fluorescence strength was well higher than the background fluorescence strength. This means that the target oligonucleotides were well fixed to the DNA chip having the complementary oligonucleotide probe.

[Example 4 - Detection of ligand by antibody-fixed chip]

(1) Preparation of antibody-fixed chip

Goat Anti-Human IgG (Jackson Immuno Research) was diluted with PBS (100, 20.4, 0.8, 0.16 ng/μL, 1μL), and was spotted on the chip prepared in the same manner as in Example 2. The spotted chip was placed in a saturated aqueous sodium chloride chamber at 25°C for 3 hours. Thus incubated chip was immersed in 1% BSA/0.05% Tween 20-PBS (PBS-T) for one hour to block the chip surface. Thus, there was prepared the antibody-fixed chip.

(2) Reaction with ligand and detection

To the antibody-fixed chip prepared in (1) above was placed closely on HybriWell (Grace Bio-Labs). In the HybriWell was placed Human 100 μL of IgG-Cy5 (Jackson Immuno Research) diluted with 1% BSA/PBS-T to have a concentration of 2 μg/mL. The chip was kept in a moisture chamber at 25°C for one our for incubation. The incubated chip was washed three times with PBS-T, rinsed with PBS, and centrifuged at 700 r.p.m. for 5 minutes. Thus dried chip was subjected to measurement of fluorescence strength by a fluorescence scanning apparatus. It was found that the portion in which the antibody was spotted in 10 ng/μL showed strength of 190, which was highly increased as compared with the background fluorescence strength.

Therefore, it was confirmed that the antibody-fixed chip prepared using the web having the reactive vinylsulfonyl group on its surface was satisfactorily used to detect a ligand reactive to the antibody fixed onto the chip.

## Claims

1. A roll of flexible web having a plurality of reactive groups on a surface thereof, in which the reactive groups are vinylsulfonyl groups that are attached to the flexible web via a linking group.

2. The roll of flexible web of claim 1, wherein the vinylsulfonyl group is represented by the following formula:

$$-SO_2-CR^1=CR^2R^3$$

in which each of $R^1$, $R^2$ and $R^3$ independently is a hydrogen atom, an alkyl group having 1 to 6 carbon atoms, an aryl group having 6 to 20 carbon atoms, or an aralkyl group having 7 to 26 carbon atoms in which its alkyl group has 1 to 6 carbon atoms.

3. The roll of flexible web of claim 2, wherein each of $R^1$, $R^2$ and $R^3$ is a hydrogen atom.

4. The roll of flexible web of claim 1, wherein the linking group contains -NH-, -S-, or -O-.

5. The roll of flexible web of claim 1, wherein the reactive group is attached to the flexible web by reacting divinylsulfone compounds with active hydrogen-containing groups which are previously provided to the flexible web.

6. The roll of flexible web of claim 5, wherein the active hydrogen-containing group is $-NH_2$, -SH, or -OH.

7. The roll of flexible web of claim 6, wherein the groups of $-NH_2$, -SH, or -OH are provided to the surface of the web

by coating the surface with polymer material having a plurality of $-NH_2$, -SH, or -OH.

**8.** The roll of flexible web of claim 6, wherein the group of $-NH_2$, -SH, or -OH are provided to the surface of the web by treating the surface with a silane-coupling agent containing a plurality of $-NH_2$, -SH, or -OH.

**9.** The roll of flexible web of claim 1, wherein a metal film or a metal oxide film is provided on the surface of the flexible web, and the metal film or metal oxide film has a plurality of -SH groups on a surface thereof.

**10.** A chip having on a surface thereof a plurality of reactive vinylsulfonyl groups attached to the surface 'via a linking group which is manufactured by dividing the roll of web of claim 1.

**11.** A process for preparing a roll of web having reactive groups on a surface thereof, which comprises bringing divinylsulfone compounds into contact with a surface of a web which has a plurality of active hydrogen-containing groups on the surface, to cause binding reaction between the hydrogen-containing group and the one vinylsulfonyl group of the divinylsulfone compound, and winding the divinylsulfone compound-bonded web to give a roll.

**12.** The process of claim 11, wherein the hydrogen-containing group is $-NH_2$, -SH or -OH.